(19)

Europäisches
Patentamt

European
Patent Office

Office européen
des brevets

(11) **EP 1 583 509 B1**

(12) **EUROPEAN PATENT SPECIFICATION**

(45) Date of publication and mention
of the grant of the patent:
**07.05.2014 Bulletin 2014/19**

(21) Application number: **03750473.5**

(22) Date of filing: **01.09.2003**

(51) Int Cl.:
**A61K 9/00** *(2006.01)*  **A61P 17/08** *(2006.01)*

(86) International application number:
**PCT/EP2003/009685**

(87) International publication number:
**WO 2004/024108 (25.03.2004 Gazette 2004/13)**

(54) **ORALLY ADMINISTRABLE COMPOSITION FOR IMPROVING HAIR AND COAT QUALITY**

ORAL VERABREICHBARE ZUSAMMENSETZUNG ZUR VERBESSERUNG DER HAAR- UND
FELLQUALITÄT

COMPOSITION A ADMINISTRATION ORALE PERMETTANT D'AMELIORER LA QUALITE DES
CHEVEUX OU DU PELAGE

(84) Designated Contracting States:
**AT BE BG CH CY CZ DE DK EE ES FI FR GB GR
HU IE IT LI LU MC NL PT RO SE SI SK TR**

(30) Priority: **09.09.2002 EP 02078706**

(43) Date of publication of application:
**12.10.2005 Bulletin 2005/41**

(73) Proprietor: **Nestec S.A.**
**1800 Vevey (CH)**

(72) Inventors:
 • **PRIDMORE-MERTEN, Sylvie**
  **CH-1005 Lausanne (CH)**
 • **LURATI, Emmanuelle**
  **CH-1260 Nyon (CH)**

 • **POURZAND-AZARMEHR, Farzaneh**
  **CH-1066 Epalinges (CH)**
 • **ROSSIO, Patricia**
  **F-06130 Grasse (FR)**
 • **DEMARCHEZ, Michel**
  **F-06620 Le Bar sur Loup (FR)**

(74) Representative: **Rupp, Christian et al**
**Mitscherlich PartmbB**
**Patent- und Rechtsanwälte**
**Sonnenstraße 33**
**80331 München (DE)**

(56) References cited:
 **WO-A-97/02041  DE-A- 19 539 859**
 **US-A- 5 626 849  US-A- 5 895 652**

## Description

The present invention relates to orally administrable compositions for improving coat or hair quality and growth.

## Background of the Invention

In recent years, there has been a progressive increase in the damage to hair or coat. Indeed, the hair perpetually undergoes a variety of stresses from the outside. That is, the hair is subjected to not only natural stresses such as ultraviolet rays from the sun, air pollution and dirt but also even more stringent stresses such as shampooing, brushing, heat from a drier, and beauty treatments such as hair-dyeing and bleaching. As a result, well-known problems of the hair are caused, including dry and rough hair, increased number of split ends, broken hair, falling hair, and reduced strength of hair fibres.

Therefore, in order to prevent or mitigate the above-described damage to the hair, various attempts have been proposed from different approaches.

Various cosmetic compositions for hair, including shampoo, hair rinse, hair treatment and the like are known. For example, the patent publication JP-A-63-105000 discloses various cosmetic compositions for hair containing an acylated peptide. US 6,251,379 provides a hair-cosmetic composition containing keratose, which is cationized with a quaternary ammonium salt, and a silicone derivative. Cationic surfactants are also frequently used for imparting smoothness to hair fibres. However, the gloss imparted thereby is not fully satisfactory, and besides, incorporation of cationic surfactants in large amounts is unfavourable in terms of safety. US 5, 895, 552 relates a method of metabolic adjuvenation and cellular repair.

Others attempts have been made through orally administrable compositions. For example, US 5,250,300 discloses a liquid internal medicine for domestic animals, which contains only an extract from stems of natural Stevia plants. It is intended for improving the physical constitution of domestic animals to further result in improvement of meat quality, milk quality and hair gloss of domestic animals.

Also, it is known that nutritional interventions may impact hair conditions, such as in WO 9856263 which discloses the combination of linoleic acid and zinc for the improvement of skin quality and coat condition in pets.

However, there is still a need in the art to provide an effective nutritional way to improve hair or coat quality, particularly shininess and hair growth in humans and animals.

## Summary of the invention

>

The invention relates to a non-therapeutic, orally administrable composition for improving hair or coat quality in humans or animals, comprising a molecule that stimulates energy metabolism of the cell and antioxidants, wherein said molecule is L-carnitine and in which the antioxidants are cysteine, vitamin C, vitamin E, and grape seed extract.

## Detailed Description of the Invention

The invention relates to a non-therapeutic, orally administrable composition for improving hair or coat quality in humans or animals, comprising a molecule that stimulates energy metabolism of the cell and antioxidants, wherein said molecule is L-carnitine and in which the antioxidants are cysteine, vitamin C, vitamin E, and grape seed extract. The following subject matter is not part of the invention:

Disclosed is an orally administrable composition tor improving hair or coat quality, which comprises as an active ingredient an effective amount of a molecule that stimulates energy metabolism of the cell, an antioxidant or combinatory admixtures thereof, in an orally acceptable carrier, is concerned.

The molecule that stimulates energy metabolism of the cell may be L-carnitine, creatine, fatty acids (mono or polyunsaturated fatty acids, particularly omega-3 fatty acids), cardiolipin, nicotinamide, carbohydrate and natural sources thereof, for example.

The amount of said molecule is of at least 1mg per kg of body weight per day, more preferably from 1mg to 1 g per kg of body weight per day.

The antioxidants are compounds that decrease protein oxidation (e.g. prevent formation of protein carbonyls). They may be sources of thiols (e.g. Lipoic acid, cysteine, cystine, methionine, S-adenosyl-methionine, taurine, glutathione and natural sources thereof), or compounds that upregulate their biosynthesis in vivo, for example. The antioxidant may also be other antioxidants such as vitamin C, vitamin E (tocopherols and tocotrienols), carotenoids (carotenes, lycopene, lutein, zeaxanthine..) ubiquinones (e.g.CoQ10), tea catechins (e.g epigallocatechin gallate), coffee extracts containing polyphenols and/or diterpenes (e.g. kawheol and cafestol), ginkgo biloba extracts, grape or grape seed extracts rich in proanthocyanidins, spice extracts (e.g. rosemary), soy extracts containing isoflavones and related phytoestrogens and other sources of flavonoids with antioxidant activity, compounds that upregulate cell antioxidant defense (e.g. ursodeoxycholic acid for increased glutathione S-transferase, ursolic acid for increased catalase, ginseng and gingenosides for increase superoxide dismutase and natural sources thereof i.e. herbal medicines), for example.

The amount of the antioxidant is of at least 0.025 mg per kg of body weight per day, more preferably from 0.025 mg to 250mg per kg of body weight per day.

The carrier may be any food or pharmaceutical product, or a nutritional supplement or a composition for oral administration. Examples for food or pharmaceuticals carriers are milk, yoghurt, curd, cheese, fermented milks, milk based fermented products, ice-creams, fermented cereal based products, milk based powders, infant formulae or tablets, liquid suspensions, dried oral supplement, wet oral supplement, dry-tube-feeding, pet food products. The composition for oral administration may be in capsules, soft capsules, tablets, pastes or pastilles, gums, or drinkable solutions or emulsions. Methods for preparing the carrier are common knowledge.

The composition may also comprise usual excipients, in particular sweeteners, flavouring agents or preservatives. It can further comprise a prebiotic and/or a probiotic micro-organism.

The compositions may be formulated according to any one of a number of techniques that are well known to this art.

Disclosed is a pharmaceutical composition containing at least one of the objective substances in an amount sufficient to achieve the desired effect in an individual can be prepared. This composition may be a tablet, a liquid, a dried oral supplement, a wet oral supplement, dry tube feeding, wet tube-feeding etc.. The pharmaceutical composition will further contain carriers and excipients that are suitable for delivering the respective active molecule of different nature to the target tissue. The kind of the carrier/excipient and the amount thereof will depend on the nature of the substance and the mode of drug delivery and/or administration contemplated. It will be appreciated that the skilled person will, based on his own knowledge select the appropriate components and galenic form to target the active compound to the skin.

Disclosed is a food composition for human consumption is prepared. This composition may be a nutritional complete formula, a dairy product, a chilled or shelf stable beverage, soup, a dietary supplement, a meal replacement, and a nutritional bar or a confectionery.

The nutritional formula is enterally administrable; for example in the form of a powder, a liquid concentrate, or a ready-to-drink beverage. If it is desired to produce a powdered nutritional formula, the homogenised mixture is transferred to a suitable drying apparatus such as a spray drier or freeze drier and converted to powder.

Disclosed is a usual food product may be enriched with the combination. For example, a fermented mills, a yoghurt, a fresh cheese, a renneted milk, a confectionery bar, breakfast cereal flakes or bars, drinks, milk powders, soy-based products, non-milk fermented products or nutritional supplements for clinical nutrition. Then, the amount of the molecule that stimulates energy metabolism is preferably of at least 50 ppm by weight and the antioxidant is preferably of at least 10 ppm by weight.

Pet food products may be prepared. The pet food formulation is a complete and nutritionally balanced pet food. It can also be a dietary supplement for pets or in the form of a pharmaceutical composition. The nutritionally complete pet food formulation may be in any suitable form, for example a powder, a dried kibble, or pellet or other dried form, extruded form, semi-moist or wet form, such as a chunk or loaf or pudding. It may be chilled or provided as a shelf stable product. This pet food may be produced by conventional methods.

Dietary adjuncts may be prepared so as to improve pet food quality. As dietary adjuncts, they may be encapsulated or may be provided in powder form and packaged in conjunction with or separately from a main meal, be it wet or dry. By way of example, a powder containing selected substances according to the invention may be packed in sachets in a powder form or in a gel or lipid or other suitable carrier. These separately packaged units may be provided together with a main meal or in multi-unit packs for use with a main meal or treat, according to user instructions.

The food composition aims to increase hair thickness and glossiness, improve hair composition and structure, modify sebum production or composition.

Disclosed is the use of an effective amount of a molecule that stimulates energy metabolism of the cell, an antioxidant or combinatory admixtures thereof, for the preparation of a composition intended to improve hair or coat quality in humans or animals.

Disclosed is a method to improve coat or hair quality in humans or animals, comprising administering to the individual, a composition as described above.

The said composition may be administered to the individual as a supplement to the normal diet or as a component of a nutritionally complete food. Disclosed is a nutritionally complete food as described above.

The amount of the composition to be consumed by the individual to obtain a beneficial effect will depend upon its size, its type, and its age. However an amount of L-carnitine of at least 1mg per kg of body weight per day and an amount of the antioxidant of at least 0.025 mg per kg of body weight per day would usually be adequate.

The following examples are given by way of illustration only and in no way should be construed as limiting the subject matter of the present application. All percentages are given by weight unless otherwise indicated.

**Examples**

**Example 1: In-vivo trials on the effect of dietary nutrients according to the present invention**

**1. MATERIAL AND METHODS**

**1.1 Study design:**

Dietary intervention was tested at 12 months of age. Mice were randomised in 6 groups of 12 mice each and fed for 3 months either diet A, B, C, D and E. Mice had free access to food and water during the whole study (ad libitum feeding) and were submitted to 12 hours light and dark cycles. Animal weight was measured once a week.

**1.2 Animals:**

Male mice C57/BL6 were obtained from Iffa credo (France) at 9 weeks of age. After 3 weeks adaptation, mice (12 weeks old) were housed individually and fed the control diet until the nutritional intervention.

**1.3 Diets:**

The control diet (diet A) composed of 18% proteins (soy and whey), 11% fat (soybean oil), 59% carbohydrates (starch + sucrose) and 10% cellulose was supplemented with active ingredients. These diets are as follows:

> **Diet A - Control:** 18% proteins (soy and whey), 11 % fat, 59% carbohydrates, and 10% cellulose.
> **Diet B:** Diet A + 0.3% L- carnitine
> **Diet C - Cocktail of antioxidants:** Diet A + 0.19% vit C, 0.03% vit E, 0.075% grape seed extract, 0.4% cysteine.
> **Diet D:** Diet A + 0.3% L- carnitine + cocktail of antioxidants of diet C.
> **Diet E:** Diet A + 0.0375% Ginkgo biloba extract (Linnea)

**1.4 Hair Sebum Lipid Measurement**

1.4.1 Hair sampling and sebum lipid extraction

Mice hairs were collected from a small area located on the back of the animal and stored at -80°C in a screw-cap tube filled with argon. Hair sebum lipids were extracted with 4 ml of heptane. Briefly, the tubes were shaken vigorously during 1 minute, centrifuged for 5 minutes at 5000 rpm before removal of the upper phase containing the lipids. The lipid phase was evaporated under nitrogen and the dried lipids finally dissolved in heptane ($1000 \mu l$) before storage at -80°C until use.

1.4.2 Qualitative analysis of hair sebum lipids

Lipids were spotted with an automatic sampler (ATS4 -Merck) and separated by one-dimensional High Performance thin Layer chromatography (HPTL) on 10x20 cm glass plates coated with silica gel. The following phase were used for lipid separation: heptane, toluene and heptane / dichoromethane / acetic acid (70/30/1). Waxes and sterol esters were separated during the first two migrations whereas polar lipids were separated during the third migration. External lipid standards were used for squalene, cholesterol esters, wax esters, triglycerides, diglycerides, linolenic acid and cholesterol.

1.4.3 Quantitative assessment of hair sebum lipids

A volume of 1 $\mu$l for each sample was injected on a Licrospher DIOL column ($5 \mu m$) and separated using heptane and heptane / isopropanol / butanol as mobiles phases before quantification on a High Performance Liquid Chromatography (HPLC) coupled to a light scattering detector (DDL31 - Eurosep). External lipid standards consisting of squalene, cholesterol esters, wax esters, triglycerides, diglycerides, free fatty acids and cholesterol were used for quantification. Data are expressed as $\mu$g of lipid per mg of hair.

**1.5 Sensorial Evaluation Of Coat Appearance**

The sensory evaluation of hair glossiness is based on information perceived by the eyes. Glossiness is assumed to depend on hair fibre properties such as guard hair straightness, density and smoothness. The gloss perception is often influenced by the colour of the evaluated sample; a dark colour appears glossier than a light colour.

A unique individual without prior training attempted a sensorial evaluation of coat gloss at sacrifice. Coat appearance was evaluated on a scale of 1 to 10 for coat shininess and greasiness, hair density and presence of white hair.

We have made an attempt to monitor coat changes through several other components that can contribute to coat appearance i.e. hair density, greasiness and presence of grey hair.

## 2. RESULTS

**2.1 Quantification of total hair sebum lipids**

2.1.1 Total lipids

Lipids values as measured by HPLC analysis were compared with one-way analyses of variance (1 w-ANOVA) for each control time, followed by a LSD-multi-comparison procedure (Least-Significant Difference) when the ANOVAs were significant at the 0.95 confidence level. Previously, within-group variances were compared by a Bartlett homogeneity test. Robust ANOVAs, involving medians and robust within-group variances were also used to confirm our statistical conclusions.

As shown in Table 1, total lipids of adult mice are significantly increased by antioxidant cocktail associated to L-carnitine. Antioxidant cocktail alone does not increase significantly total lipid when compared to the control mice (Diet A). Diet E (Gingko) and B (L-carnitine) do not induce significant changes.

Table 1: Total lipids values as measured by HPLC analysis were analyzed after transformation into logarithms in order to reduce the dispersion of the variances (Bartlett's test) number of mice were n>10. Results were expressed as $\mu$g of lipids per mg of hair.

| Diets | Median | SD (Sn) | Anova |
|---|---|---|---|
| **A: CONTROL** | 1.81 | 0.74 | ns |
| **B: L-Carnitine** | 1.70 | 0.44 | ns |
| **C: Antioxidants** | 2.51 | 0.82 | ns |
| **D: Antioxidants+ L-carnitine** | **3.18** | 1.42 | p<0.05 |
| **E: Ginkgo** | 1.82 | 0.62 | ns |

2.1.2 Total lipid composition

The increase of total lipids that is observed in mice fed diets containing the antioxidant cocktail alone or associated to L-carnitine is mainly due to changes in the amount of the polar and non-polar lipids (see below in Table 2A).

Table 2A: Amount of lipids for each dietary group was determined by HPLC and grouped into 3 classes i.e. non-polar lipids (esters of cholesterol and wax), triglycerides and polar lipids (cholesterol, diglycerides and fatty acid). Results are expressed as median and $\mu$g of lipids per mg of hair. Significant values (p< 0.05) are in bold.

| Diets | Non polar lipids | Triglycerides | Polar lipids |
|---|---|---|---|
| **A: CONTROL** | 0.90 | 0.68 | 0.19 |
| **B: L-Carnitine** | 0.96 | 0.63 | 0.18 |
| **C: Antioxidants** | **1.55** | 0.60 | **0.25** |
| **D: Antioxidants+ L-carnitine** | **1.88** | 0.85 | **0.27** |
| **E: Ginkgo** | 1.05 | 0.60 | 0.19 |

2.1.3 Non-polar lipids

The mice fed with diets supplemented with the antioxidant cocktail alone or combined with L-carnitine (diets C and D) had a significantly higher amount of non-polar lipids when compared to all other groups (Table 2A).

2.1.4 Triglycerides

The triglycerides present in the sebum were not significantly different between groups. (Table 2A).

2.1.5 Polar lipids

The polar lipids (cholesterol, diglycerides and fatty acids) were significantly higher in mice fed antioxidants alone or combined to L-carnitine (diet C and D) (Table 2A).

**2.2 Hair sebum lipid balance**

HPLC analysis of hair sebum composition of mice fed the control diet (A) provided data for lipid balance that were consistent with those reported in the literature i.e. 46% non-polar lipids (cholesterol and wax esters), 46% triglycerides and 8% of polar lipids (cholesterol, diglycerides and fatty acid (Table 2B).

Table 2B: determination of hair lipid balance for each dietary group. Lipid amounts were determined by HPLC and grouped into 3 classes i.e. non-polar lipids (esters of cholesterol and wax), triglycerides and polar lipids (cholesterol, diglycerides and fatty acid). Results are presented in percent of total lipids.

| DIETS | Percent of total lipids | | |
|---|---|---|---|
| | Non polar lipids | Triglycerides | Polar lipids |
| **A: CONTROL** | 46 | 46 | 8 |
| **B: L-Carnitine** | 55 | 35 | 10 |
| **C: Antioxidants** | 61 | 31 | 8 |
| **D: Antioxidants+ L-carnitine** | 62 | 30 | 8 |
| **E: Ginkgo** | 51 | 41 | 8 |

Diets containing the antioxidant cocktail alone (diet C) or combined with L-carnitine (diet D) increased the proportion of non-polar lipids to about 60% in hair sebum as compared to 46% in the control (Table 2B). Diets supplemented with Gingko (diet E) or L-carnitine (diet B) had little effect on lipid balance when compared to the control diet (A).
Analysis of sebum lipids by HPTLC shows that squalenes were absent in hair sebum of mice (Data not shown).

**2.3 Sensorial evaluation of the coat appearance at sacrifice: shininess, hair density, greasiness and presence of grey hair**

In general, sensorial evaluation did not show significant differences in coat shininess except for mice fed with antioxidants (Diet C) that were glossier than all others groups and Ginkgo (diet E) that came out shinier than the control mice (diet A). Grey hair appeared to be more abundant in mice fed antioxidants associated to L-carnitine (diet D).

**3. CONCLUSION**

Mice fed diet containing the cocktail of antioxidants associated or not to L-carnitine (diet D and diet C respectively) showed an increase in non-polar (cholesterol esters and wax esters), and polar lipids (Fatty acids, diglycerides and cholesterol) as well as total lipids in hair sebum when compared to the control diet (A).
A modification of the lipid balance was also observed for these 2 diets with an increase proportion of non-polar lipids up to 60 % as compared to 46% in the control diet.
Sensory evaluation gave best scores for mice fed the diet supplemented with the cocktail of antioxidants alone (vit C, vit E, grape seed extract and cysteine) as compared to mice fed all other diets. Interestingly, the diet combining the cocktail of antioxidants and L-carnitine (diet D) also induced similar lipid changes in the sebum but did not score well for shininess. One of the possible reason for this difference is the presence of greater amount of grey hair in mice fed this diet since it has been previously demonstrated that gloss is best assess on a black color. There is presently no explanation for the increased amount of grey hair induced by the diet D, however we could postulate that this is a transient defect in melanogenesis since long term feeding with diet D (20 months) does not induce such a phenotype.

**Example 2:**

**1. MATERIAL AND METHODS**

**1.1 Animal Study Design**

The effect of long term nutritional interventions with diets A, B was tested in C57/BL6 male mice obtained from Iffa credo (France) at 9 weeks of age. During the study, mice were housed individually; submitted to 12 hours light and dark cycles and had free access to food and water.

The effect of nutritional interventions was tested at different time during the intervention i.e. in young (6 months of age), adult (12 and 18 months of age) and old mice (21 and 24 months of age) that is after 3, 9, 15, 18 and 21 months of treatment. At the beginning of the intervention mice were randomised in groups of 12 mice and fed ad libitum. Animal weights and food consumption was recorded weekly for each animal.

**1.2 Diets**

The control diet (diet A) composed of 18% proteins (soy and whey), 11% fat, 59% carbohydrates and 10% cellulose was supplemented a cocktail of antioxidants comprising vit C, vit E, grape seed extract and cysteine and L-Carnitine (diet B).

**1.3 Coat Gloss Measurement**

Our objective was to evaluate the dorsal surface of mice coat by means of optical variables with non-destructive methods and relate these to the visual grades of glossiness. We have used 2 different instrumental technologies and compared their results to sensorial evaluation.

**1.3.1 Gloss Measurement with Macbeth ColorEye XTH**

Coat gloss was measured by reflectrometry using a portable Spectrophotometer "Macbeth, Colour-eye XTH" equipped with a CIE (Commission International de l'Eclairage) standard daylight illumination source $D_{65}$, a 10 mm circular diameter of view (RAV), in both Specular Component Excluded (SCE) and Specular Component Included (SCI) (This Measurement includes the specular component and minimises all aspects of appearance other than colour ($\rightarrow$colour)). Coat gloss was calculated as the L*a*b* (Colour parameters) colour differences between SCI and SCE mode according to following equation.

Equation 1

$$\Delta E^*_{SCI-SCE} = Gloss = \sqrt{(L^*_{SCI} - L^*_{SCE})^2 + (a^*_{SCI} - a^*_{SCE})^2 + (a^*_{SCI} - a^*_{SCE})^2}$$

By excluding the colour of the measurements, □E* (SCI-SCE) provides an evaluation of the gloss of the sample. These calculations were used to measure the glossy aspects of pet fur. The Macbeth CE-XTH is a spectrophotometer and the gloss values resulted by this methodology can be influenced by the colour of the sample, in spite of the use of an equation to eliminate this effect.

Coat gloss was measured on live mice on the lowest part of the back near the tail. Three measurements were performed on each animal

**1.3.1 Gloss by sensory**

The sensory evaluation of hair glossiness is based on information perceived by the eyes. Glossiness is assumed to depend on hair fibre properties such as guard hair straightness, density and smoothness. The gloss perception is often influenced by the colour of the evaluated sample; a dark colour appears glossier than a light colour.

A unique individual without prior training attempted a sensorial evaluation of coat gloss at sacrifice. Coat appearance was evaluated on a scale of 1 to 10 for coat shininess and greasiness, hair density and presence of white hair.

## 2. RESULTS

### 2.1 Coat Gloss Measurements With Macbeth XTH

**Effect of diet on coat gloss**

Mice coat gloss evaluation was done after 15 months of supplementation (mice of 18 months of age). The results are showed in table 3.

Table 3: Shows the median of coat gloss values as measured by the Macbeth XTH spectrophotometers. Values are given in gloss units.

| Diet | Median of Gloss values |
|---|---|
| Control (A) | 0.515 |
| Carnitine + Antioxidant (B) | 0.830 |

Coat gloss values for each mouse were normalized against the average values observed for the control diet in 18 months old mice (data not shown). Analysis of variance ANOVA was used to test the significance of the difference between treatments at 95% of confidence. The group "Camitine + Antioxidant" (B) has a more glossy coat (P-value of 0.00023) after 15 months of supplementation when compared to the control group (A).

### 2.2 Sensorial evaluation of the coat appearance at sacrifice: shininess, hair density, greasiness and presence of grey hair

Hair density, greasiness and the presence of grey hair were investigated separately and a correlation matrix was calculated to determine relationships between the different parameters.

When all animals of all dietary groups were considered, hair density was positively correlated to coat shininess. Meaning that, the density of mice hair, influence the decision of the panellists. Grey hair however was negatively correlated with sensorial evaluation of coat gloss for 18 months old mice but any correlation was calculated for 24 months old mice (Table 4). No significant correlation was found for the effect of hair greasiness on coat gloss.

Table 4: Correlation between sensorial parameters. The significant correlation coefficient was calculated for n (number of measurements) = 60; p = 5%, r >0.25. (Table of Fisher &Yates). NS = Non-significant relationship was found.

| | Gloss sensory | |
|---|---|---|
| | 18 months age | 24 months age |
| Hair density | + | + |
| Grey hair | - | NS |
| Greasiness | NS | NS |

### Example 3 : In-vivo trial on dogs

Resistance to epilation was measured on dogs. A diet is prepared containing about 4.6% tallow bleach, 66% corn yellow, 16% poultry, 9% beef & bone meal, 4% corn germ, 2% corn gluten meal to which is added a cocktail of bioactive ingredients composed of 0.036% Vitamin E, 0.08% Grape Seed Extract, 0.1% vit C, 35% and 0.1 % L-Carnitine.

We have found that coat hair of dog fed the above diet show an increase resistance to depilation compared to the dogs fed the same diet without the cocktail of bioactive ingredients.

### Example 4: Dry pet food

A feed mixture is made up of about 58% by weight of corn, about 5.5% by weight of corn gluten, about 22% by weight of chicken meal, 2,5% dried chicory, 0.1% carnitine, 0.1% Vit C, vit E (150 IU / kg), 0.05% grape seed proanthocyanidin extract and 0.4% cysteine as antioxidant, salts, vitamins and minerals making up the remainder. The fed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food is able to improve coat quality in dogs, particularly coat gloss.

### Example 5: Dry pet food

A feed mixture is prepared as in example 4, using 0.2% carnitine and 0.05% ginkgo biloba extract as antioxidant. Then, the fed mixture is processed as in example 4. The dry dog food is particularly intended to improve coat quality in dogs, particularly coat gloss.

### Example 6: Nutritional formula

A nutritional composition is prepared, and which contains for 100 g of powder: 15 % of protein hydrolysate, 25 % of fats, 55 % carbohydrates (including maltodextrin 37 %, starch 6 %, sucrose 12 %), traces of vitamins and oligoelements to meet daily requirements, 2 % minerals and 3 % moisture and 2% pyruvate and 1% carnosine or carnosine precursor as antioxidant.

13 g of this powder is mixed in 100 ml of water. The obtained formula is particularly intended for improving hair growth and hair quality, in particular shininess.

### Claims

1. A non-therapeutic, orally administrable composition for improving hair or coat quality in humans or animals, comprising a molecule that stimulates energy metabolism of the cell and antioxidants, wherein said molecule is L-carnitine and in which the antioxidants are cysteine, vitamin C, vitamin E, and grape seed extract.

### Patentansprüche

1. Nicht therapeutische, oral verabreichbare Zusammensetzung zur Verbesserung der Haar- oder Fell- bzw. Hautqualität bei Mensch oder Tier, aufweisend ein Molekül, dass den Energiemetabolismus der Zellen anregt, und Antioxidantien, wobei das Molekül L-Carnitin und die Antioxidantien Cystein, Vitamin C, Vitamin E und Traubenkernextrakt sind.

### Revendications

1. Composition non thérapeutique apte à être administrée oralement pour améliorer la qualité des cheveux ou du pelage/des poils chez les humains ou les animaux, comprenant une molécule qui stimule le métabolisme énergétique de la cellule et des antioxydants, dans laquelle ladite molécule est de la L-carnitine et dans laquelle les antioxydants sont de la cystéine, de la vitamine C, de la vitamine E et de l'extrait de pépins de raisins.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63105000 A
- US 6251379 B
- US 5895552 A
- US 5250300 A
- WO 9856263 A